# FASCICULE DE BREVET EUROPEEN

(11) **EP 3 109 327 B1**
(45) Date de publication et mention de la délivrance du brevet: **12.06.2019**
(21) Numéro de dépôt: 16176066.5
(22) Date de dépôt: 23.06.2016
(51) Int. Cl.: C12Q 1/68

(54) **KIT DE PCR POUR L'ÉLECTROPHORÈSE CAPILLAIRE**
PCR KIT FÜR KAPILLARELEKTROPHORESE
PCR KIT FOR CAPILLARY ELECTROPHORESIS

(30) Priorité: 23.06.2015 FR 1555745
(43) Date de publication de la demande: 28.12.2016
(73) Titulaire: Idemia Identity & Security France, 92400 Courbevoie (FR)
(72) Inventeur: RAGOT, Marcelin, 92130 Issy Les Moulineaux (FR); TONSON, Benoît, 92130 Issy Les Moulineaux (FR); POUET, Marina, 92130 Issy Les Moulineaux (FR); ALOS, Rafael, 92130 Issy Les Moulineaux (FR); FORTIN, Nathalie, 92400 Courbevoie (FR)
(74) Mandataire: Regimbeau

(56) Documents cités:
- EP-A2- 0 414 469
- US-A1- 2003 235 827

## Description

La présente invention concerne un kit de PCR permettant d'améliorer la détection de motifs particuliers d'acides nucléiques et d'augmenter la fiabilité des résultats obtenus.

Plus précisément, l'invention propose d'utiliser des amorces modifiées ayant des caractéristiques particulières permettant, lors d'un traitement d'électrophorèse, de mettre en évidence, sur les échantillons contenant des acides nucléiques, l'équivalent d'un codage caractéristique de motifs particuliers d'acides nucléiques, ce codage étant tel qu'il permet de mieux détecter la présence de ces motifs.

L'invention propose également un procédé de détection de motifs d'acides nucléiques d'intérêt (par exemple STR, mini-STR ou VNTR) utilisant de telles amorces.

Elle trouve avantageusement - mais non limitativement - application dans le domaine de l'électrophorèse capillaire.

### DOMAINE TECHNIQUE GENERAL ET ART ANTERIEUR

L'électrophorèse capillaire en gel est aujourd'hui largement utilisée pour obtenir des profils ADN permettant de détecter des **variations alléliques** et donc de **différencier les individus** (*Butler,J.M (1995)*)*.*

Selon le NIST (http://www.cstl.nist.gov/strbase/str_fact.htm), le nombre d'allèles détectables par un kit de PCR classique aux Etats-Unis (i.e., contenant les marqueurs génétiques obligatoires aux États-Unis) est de 425 allèles (sur les loci : CSF1PO, FGA, TH01, TPOX, VWA, D3S1358, D5S818, D7S820, D8S1179, D13S317, D16S539, D18S51, D21S11). En outre, le nombre d'allèles à détecter pour un kit de PCR classiquement utilisé en Europe et pouvant être utilisé aux Etats-Unis (i.e., contenant les marqueurs obligatoires Européens et américains) est de 552 allèles (sur les loci cités ci-dessus, auxquels s'ajoutent les marqueurs européens : D1S1656, D2S441, D2S1338, D10S1248, D12S391, D19S443, D22S1045, SE33).

Plus précisément, les individus sont aujourd'hui distinguables par la taille de petites séquences répétées appelée « short tandem repeats » (STR), qui sont retrouvées dans les portions non-codantes de nombreux loci. Il existe de nombreux kits de PCR multiplex ciblant ces STR (voir par exemple le lien suivant : http://www.cstl.nist.gov/strbase/multiplx.htm). Classiquement, les échantillons ADN testés subissent une PCR et le produit de cette PCR (i.e., les amplificats générés) est ensuite analysé par électrophorèse capillaire. Chacun des allèles contenus dans l'échantillon contient un nombre précis de STR, caractéristique de l'individu, qui influe sur la taille de l'amplificat généré par la PCR. Chaque amplificat a un marquage et une taille donnée, détectable par un pic sur l'électrophorégramme correspondant. La combinaison de ces pics renseigne sur l'identité de l'individu.

**L'électrophorégramme** est le résultat issu de l'électrophorèse capillaire, laquelle associe une donnée analogique lue sur un capteur CCD à une quantité de matière (rendue détectable par amplification et par marquage à l'aide d'un ou de fluorochrome(s)). Plus la valeur mesurée par le capteur CCD est grande (jusqu'à la limite de saturation), plus la probabilité que le signal révèle la présence d'un acide nucléique d'intérêt est forte, et donc plus l'information est fiable.

Cet électrophorégramme est malheureusement sujet à diverses variations de qualité en raison principalement :
- De la présence d'artefacts générant l'apparition de faux pics sur le signal détecté, ces artefacts pouvant être dus :
   ∘ A des mauvais fonctionnements de la polymérase. Deux phénomènes sont connus :
      ▪ Une séquence répétitive en moins sur un STR (connus sous le nom de « Polymerase stuttering ») ;
      ▪ A l'addition d'un nucléotide A en fin de copiage (connu sous le nom d'addition A ou phénomène d'épaulement sur les profils obtenus,
   ∘ Au bruit de fond numérique/analogique (lié au capteur CCD, bruit du capteur, lumière parasite, perturbations optiques liées à la chaleur, vibrations, capteur peu performant, etc.), qui génère un rapport signal / bruit fluctuant, et
   ∘ A des phénomènes de saturation (ou « Pull-up », dus à une mauvaise calibration du capteur CCD, à une mauvaise séparation des canaux de couleur, un capteur peu performant).
   ∘ Aux fluorochromes restés libres et injectés dans le capillaire (connu sous le nom de « Dye blob »)
   ∘ Aux perturbations électriques influant sur l'électrophorèse capillaire (connu sous le nom de « spike »)
- De la perte de résolution en fin d'électrophorégramme (due à l'étalement de la matière dans le capillaire).
- De l'absence de certains pics dûe à un problème d'amplification (PCR) lié à des amorces positionnées sur une zone de l'ADN mutagène (phénomène connu sous le nom de « allele drop-out »

Aujourd'hui, la nature analogique de la donnée mesurée par le capteur et les divers paramètres perturbateurs entrant en jeu lors de la construction d'un électrophorégramme ne permettent pas de s'affranchir d'une **relecture via une personne physique experte** du domaine technique, qui doit, grâce à ses connaissances générales et à son expérience en la matière, certifier la qualité d'un profil. En général, l'expert se base sur l'amplitude des données et sur la forme de celles-ci (un signal valide présente une forme particulière, connue de l'homme du métier, à cause du phénomène de perte de résolution tout au long de l'éléctrophorégramme, s'accentuant vers la fin) pour valider l'extraction d'un profil. Ainsi, la méthode actuelle d'analyse des résultats ne peut pas être automatisée, car elle ne revêt pas un caractère totalement objectif. De plus, il est impossible de chiffrer avec quelle probabilité la donnée sélectionnée par l'expert est proche de la réalité.

### PRESENTATION GENERALE DE L'INVENTION

Un but général de l'invention est de proposer une méthode plus fiable et plus objective pour détecter la présence de motifs d'acides nucléiques d'intérêt dans un échantillon, à partir d'une information lue sur un électrophorégramme. L'invention esr décrite dans les revendications ci-jointes.

Plus précisément, l'invention propose une méthode de lecture d'un profil d'acides nucléiques très robuste aux erreurs dans le signal (élimination des phénomènes stutter, épaulements, pull-up,...), et moins sensible au bruit de fond analogique, ce qui assure une très forte probabilité objective de chaque information lue sur un électrophorégramme. Cette méthode s'affranchit d'une relecture par un expert et est donc automatisable.

Pour ce faire, la méthode de l'invention nécessite l'utilisation de groupes d'amorces spécifiques du motif recherché, ci-après nommé **n-AMC.**

Ces groupes d'amorces n-AMC sont avantageusement intégrées dans un **kit de PCR dit « codant »,** qui est également un objet de l'invention. Ce kit de PCR permet de « coder » chaque motif recherché sur plusieurs variables (taille de base, couleur), de façon très spécifique (cf. exemples ci-dessous). Ce kit est notamment utilisable pour détecter des Short Tandem Repeat (ou STR) par exemple les STRs ayant une taille comprise entre 50 et 450 paires de bases (pb). Ce kit est donc utilisable en particulier en sciences forensiques.

La présente invention vise également une méthode de détection de motifs d'intérêt dans un échantillon, utilisant de telles amorces ou un tel kit.

La présente description vise également une méthode de lecture d'un électrophorégramme produit par un tel kit.

Enfin, la présente description fournit des préconisations pour utiliser ces méthodes, plus particulièrement concernant le paramétrage des amorces de l'invention.

### Description des groupes d'amorces de l'invention (n-AMC)

Au sens de l'invention, un « n-AMC » est un groupe d'amorces différenciables permettant d'amplifier spécifiquement un motif d'intérêt à détecter sur un acide nucléique. Le chiffre « n » dans « n-AMC » indique le nombre de couples d'amorces spécifiques dudit motif contenus dans ledit groupe. Ainsi, un « 3-AMC » contient trois couples d'amorces permettant d'amplifier spécifiquement un même motif, un « 4-AMC » contient quatre couples d'amorces permettant d'amplifier spécifiquement un même motif, etc.

Par « **acide nucléique** », on entend, au sens de la présente invention, une séquence nucléique ou d'acides nucléiques, un polynucléotide, un oligonucléotide, une séquence de polynucléotides, ou encore une séquence nucléotidique, termes qui seront employés ici indifféremment. Par ce terme, on entend désigner un enchaînement précis de nucléotides, comportant (ou non) des nucléotides non naturels, et pouvant correspondre aussi bien à un ADN double-, un ADN simple-brin ou encore à des produits de transcription desdits ADNs. De préférence, il s'agit d'un ADN simple-brin ou double-brin.

Comme indiqué ci-dessus, chaque n-AMC contient plusieurs couples d'amorces spécifiques d'un même motif d'intérêt (appelé ci-après « motif A »).

Par « **motif d'intérêt**», on entend désigner une région d'un acide nucléique contenant un fragment nucléotidique caractéristique que l'on cherche à détecter et/ou à identifier. Ledit fragment peut par exemple être un enchainement caractéristique de certaines bases. Alternativement, ledit fragment peut être une répétition de séquences caractéristiques, également appelées séquences répétées, (telles que les « short tandem repeats » ou « STR », mini-STRs ou VNTRs), dont on cherche à mesurer le nombre. Enfin, ledit fragment peut avoir subi une insertion de nucléotides ou, au contraire, une délétion de nucléotides, par rapport à une population contrôle. Ces motifs d'intérêt sont de préférence les mêmes que ceux détectés par les kits commerciaux (PowerPlex, etc.). Dans le cadre de la présente invention, ledit fragment est de préférence un STR allélique, qui comme expliqué plus haut, permet de différencier des individus en sciences forensiques.

Par « **amorces** », on entend ici un fragment nucléotidique comprenant par exemple de 15 à 40 nucléotides, notamment de 18 à 30 nucléotides, possédant une spécificité d'hybridation dans des conditions déterminées pour former un complexe d'hybridation avec une séquence d'acide nucléique cible, par exemple située à l'extrémité d'un motif d'intérêt tel que défini ci-dessus. Ces amorces sont de préférence des ADN simple-brin. Pour réaliser une amplification par PCR, il est nécessaire d'utiliser ces amorces par « couple », l'une des amorces du couple s'hybridant en amont de la région d'intérêt à amplifier, l'autre en aval.

Par « **couple d'amorces spécifiques d'un motif A**», on entend ici un couple d'amorces « permettant d'amplifier spécifiquement un motif A». En pratique, il s'agit de deux amorces s'hybridant spécifiquement de part et d'autres du motif d'intérêt, les deux régions d'hybridation étant distantes d'au maximum 2000 paires de bases (Butler M. et al, Fundamentals of Forensic DNA typing, 2009). Par « **spécifiquement** », on entend que l'hybridation desdites amorces n'a pas lieu sur un acide nucléique ayant une séquence possédant moins de 80% d'homologie avec la région visée. Les paramètres définissant les conditions de stringence adéquates dépendent de la température à laquelle 50% des brins appariés se séparent (Tm). Pour les séquences de longueur inférieure à 30 bases, Tm est définie par la relation : Tm= 4 (G+C) + 2 (A+T). Dans des conditions de stringence adéquates (i.e., dans lesquelles les séquences aspécifiques n'hybrident pas), la température d'hybridation est de préférence de 5 à 10°C en dessous de Tm, et les tampons d'hybridation utilisés sont de préférence des solutions de force ionique élevée (telle qu'une solution 6xSSC par exemple). L'homme du métier est tout à fait capable d'identifier des amorces spécifiques d'un fragment nucléotidique donné, une fois la séquence de celui-ci connue. Dans les sciences forensiques, des couples d'amorces permettant de se fixer de part et d'autres des régions contenant des STR, permettant ainsi de générer des amplificats distincts en fonction des individus, sont bien connus de l'homme du métier (cf. la liste proposée sur le lien : http://www.cstl.nist.gov/strbase/primer.htm).

Dans le cadre de l'invention, il est essentiel qu'au sein de chaque n-AMC, au moins deux couples d'amorces spécifiques d'un même motif génèrent des amplificats différenciables.

Par « **amplificat** », on entend ici un fragment nucléotidique produit par une réaction de polymérisation ayant eu lieu dans l'échantillon testé en présence des amorces oligonucléotidiques spécifiques du motif d'intérêt, dans les conditions de polymérisation appropriées. Cet amplificat a quasiment la même séquence nucléotidique que le motif d'intérêt (à ceci près que ses extrémités sont constituées des amorces utilisées pour le générer et d'éventuelles régions flanquantes qui entourent la région d'intérêt).

Par « **différenciable** » , on entend ici qu'il est possible de différencier chaque amplificat sur la base d'une caractéristique objective de celui-ci, détectable par les techniques actuelles de séparation des acides nucléiques, par exemple par électrophorèse (sur gel d'agarose ou de polyacrylamide, éventuellement capillaire) ou par chromatographie. Cette caractéristique est sa taille, son marquage, etc.

Ainsi, chaque n-AMC contient, pour chaque motif d'intérêt, au moins deux couples d'amorces dont :
- un couple d'amorces spécifiques du motif A, produisant un amplificat de taille T, détectable lors de l'électrophorèse avec une couleur C. Ce couple d'amorces sera ci-après défini comme le couple d'amorces « **de référence** » de l'n-AMC pour le motif A. Il s'agit de préférence du couple d'amorces classiquement utilisé dans les kits et méthodes de l'art antérieur pour détecter le motif A, et
- au moins un autre couple d'amorces spécifiques du motif A, générant un amplificat différenciable de celui produit par le couple de référence. Cet autre couple d'amorces est ci-après nommé couple d'« **amorces modifiées** ».

Dans un mode de réalisation préféré, les amplificats générés par ces deux couples d'amorces se distinguent soit par leur taille (au moins une base de différence), soit par leur marquage soit par leur taille et leur marquage.

Dans un mode de réalisation encore plus préféré, le couple d'amorces modifiées génère un amplificat qui contient une base de plus ou de moins par rapport à celui généré par les amorces de référence, ou génère un amplificat qui est détectable par un signal différent de celui porté par l'amplificat généré par les amorces de référence (par exemple une couleur C' distincte de C).

### Kit de l'invention

Dans un premier aspect, la présente invention concerne un kit de PCR contenant, outre les réactifs classiquement utilisés dans une PCR (dNTPs, Taq polymérase, etc.), un n-AMC tel que défini ci-dessus, dans lequel n est supérieur ou égal à 2.

Plus précisément, la présente invention vise un kit de PCR contenant, pour chaque motif d'intérêt à identifier sur un acide nucléique, au moins deux couples d'amorces spécifiques dudit motif, lesdits couples générant, pour chaque motif, au moins deux amplificats différenciables.

De cette façon, lors de la mise en oeuvre de l'électrophorèse capillaire, on dispose d'une redondance dans l'information de détection du motif (en particulier pour n=2). Transposé dans le domaine numérique, c'est l'équivalent d'un codage avec redondance et code correcteur (notamment pour n>2).

Selon un mode de réalisation, kit de PCR contenant, pour chaque motif d'intérêt à identifier sur un acide nucléique, au moins deux couples d'amorces spécifiques dudit motif, lesdits couples étant adaptés pour générer, pour chaque motif, au moins deux amplificats différenciables. Dans un mode de réalisation préféré, ledit kit contient un 2-AMC, c'est-à-dire deux couples d'amorces spécifiques de chaque motif à détecter, lesdits couples générant, pour chaque motif à détecter, deux amplificats différenciables.

Dans un mode de réalisation préféré, ledit kit contient un 3-AMC, c'est-à-dire trois couples d'amorces spécifiques de chaque motif à détecter, lesdits couples générant, pour chaque motif à détecter, trois amplificats différenciables.

De préférence, lesdits amplificats sont différenciables soit par leur taille, soit par leur marquage, soit par une combinaison de ces caractéristiques.

De manière encore plus préférée, lesdits couples d'amorces génèrent des amplificats dont la taille diffère d'au moins une base et/ou génèrent des amplificats marqués différemment.

Il est en particulier possible d'utiliser des couples d'amorces modifiées générant des amplificats dont la taille diffère d'une paire de bases, de deux paires de bases, de trois paires de bases, voire de quatre paires de bases, ou d'un nombre quelconque de paires de bases.

Selon un mode de réalisation, le kit de PCR contient au moins deux couples d'amorces spécifiques à un motif d'intérêt à identifier sur un acide nucléique, lesdits couples étant adaptés pour générer, pour ledit motif, au moins deux amplificats différenciables par leur taille et dont la taille diffère d'une paire de bases, de deux paires de bases, de trois paires de bases, ou de quatre paires de bases.

Il est en outre possible d'utiliser des couples d'amorces modifiées générant des amplificats marqués différemment. Les « **marqueurs** » portés par les amplificats peuvent être des isotopes radioactifs, des enzymes (notamment une peroxydase ou une phosphatase alcaline), des composés chimiques chromophores, des composés chromogènes, fluorigènes ou luminescents, des analogues de base nucléotitiques, ou encore des ligands tels que la biotine ou tout autre moyen équivalent. Parmi les isotopes radioactifs utilisés, on peut citer le ³²P, le ³³P, le ³⁵S, le ³H ou le ¹²⁵I. Les entités non radioactives sont sélectionnées parmi les ligands tels la biotine, l'avidine, la streptavidine, la dioxygénine, les haptènes, les colorants, les agents luminescents tels que les agents radioluminescents, chémiluminescents, bioluminescents, fluorescents, ou phosphorescents. De préférence, les amplificats générés par la PCR portent des marqueurs fluorescents détectables par électrophorèse capillaire tels que la fluorescéine, carboxy-fluorescéine, le texas red, Rhodamine-Red, carboxy-X Rhodamine (CXR), la Cyanine, les Alexa fluor, ou tout autre cité sur le site http://fr.wikipedia.org/wiki/Fluorochrome. Il est notamment possible d'utiliser les marqueurs décrits dans Butler M. et al, Fundamentals of Forensic DNA typing, 2009, en particulier le 5-FAM 5-carboxy fluorescein, la JOE 6-carboxy-2,7-dimetoxy-4 ,5-dichlorofluorescein, la ROX (CXR) 6-carboxy-X-rhodamine, la Fluorescein TMR (TAMRA), la N,N,N _,N -tetramethyl-6-carboxyrhodamine, la TET 4,7,2 ,7-tetrachloro-6-carboxyfluoresceine, ou encore la HEX 4,7,2 ,4 ,5 ,7 -hexachloro-6-carboxyfluoresceine.

De tels amplificats peuvent être obtenus en utilisant des amorces particulières, par exemple des amorces dont la taille diffère ou portant un marqueur différent.

Il est en effet possible de contrôler très précisément la taille d'un amplificat obtenu par PCR en modulant directement la taille des amorces utilisées. Notamment, il est possible de générer des amplificats dont la taille diffère de X paire(s) de bases en utilisant des amorces modifiées contenant X bases de plus ou de moins par rapport aux amorces de référence. Plus précisément, il est possible d'obtenir deux amplificats ayant X paire(s) de base de différence en utilisant deux couples d'amorces identiques à X base(s) près (X base(s) de plus ou de moins dans une des amorces du couple d'amorces modifiées par rapport à l'amorce équivalente dans le couple d'amorces de référence).

Dans un mode de réalisation préféré, le kit de l'invention contient des amorces modifiées dont la séquence nucléotidique est identique à celle des amorces de référence, à X base(s) près. De préférence, X est un entier compris entre 1 et 10.

Il est également possible de générer des amplificats de taille différente en utilisant des amorces dont la région d'hybridation sur l'acide nucléique d'intérêt est décalée (de X bases) par rapport à celle des amorces de référence. De préférence, X est un entier compris entre 1 et 10.

En outre, il est possible de contrôler très précisément la nature du marqueur attaché à un amplificat en utilisant des amorces elles-mêmes marquées. Plus précisément, il est possible d'obtenir deux amplificats différenciables par le marqueur qu'ils portent en utilisant deux couples d'amorces portant un marqueur différent (par exemple un fluorochrome différent).

Dans un autre mode de réalisation préféré, le kit de l'invention contient donc un couple d'amorces modifiées dont au moins une amorce porte un marqueur différent du ou des marqueur(s) porté(s) par les amorces de référence.

Outre le n-AMC, le kit de PCR de l'invention peut contenir tout réactif utilisable pour réaliser une PCR, à savoir une polymérase Taq recombinante, un tampon pour maintenir le pH stable dans le milieu réactionnel (typiquement du Tris-HCl), des dNTPs, du MgCl2, etc. Les conditions d'amplification d'un acide nucléique et les réactifs à utiliser à cet effet sont bien connus de l'homme du métier. Il est possible à ce sujet de consulter des ouvrages de biologie moléculaire, tels que Sambrook et al. (Molecular cloning: a laboratory manual, Cold Spring Harbor Laboratory; 3rd edition, 2001).

Selon un mode de réalisation, le kit contient un contenant distinct pour chaque amorce dudit kit. Dans ce mode de réalisation, chaque amorce est disposée dans un contenant distinct au sein du kit.

Selon un autre mode de réalisation, le kit contient un contenant distinct pour chaque couple d'amorces dudit kit. Dans ce mode de réalisation, les amorces de chaque couple d'amorces du kit sont pré-mélangées deux à deux dans un contenant distinct pour chaque couple d'amorces.

Selon un autre mode de réalisation, le kit contient un contenant unique pour tous les couples d'amorces dudit kit. Dans ce mode de réalisation, les amorces de chaque couple d'amorces du kit sont pré-mélangées dans un contenant unique.

Par « **contenant** », on entend ici un récipient, un tube, une ampoule, une seringue, une cartouche, une bouteille, un flacon, une fiole, une boite, une capsule, une enveloppe, un puits, une alvéole, un sac, un sachet, ou un patch. Le contenant peut être hermétique ou non hermétique, fermé par exemple par un bouchon, une capsule, un opercule, un film, ou non fermé.

### Pour n=2, le kit de l'invention contient un 2-AMC.

Dans ce mode de réalisation particulier, le kit de l'invention contient un couple d'amorces de référence capable d'amplifier spécifiquement le motif A, produisant un amplificat de taille T (T paires de base), marqué avec un marqueur M (par exemple un fluorochrome de couleur C), et :
- un couple d'amorces modifiées capable d'amplifier spécifiquement le motif A, produisant un amplificat ayant une taille T' différente de T, ledit amplificat étant marqué avec un marqueur M (par exemple un fluorochrome de couleur C),
   ou
- un couple d'amorces modifiées capable d'amplifier spécifiquement le motif A, produisant un amplificat ayant une taille T, ledit amplificat étant marqué avec un marqueur M' différent de M (par exemple un fluorochrome de couleur C' différente de C).

De préférence, la taille T' de l'amplificat diffère de T d'une, de deux, de trois, voire de quatre paires de bases (en plus ou en moins).

Dans le cas plus particulier où T' diffère de T d'une paire de bases, le kit de l'invention contient :
- Un couple d'amorces de référence capable d'amplifier spécifiquement le motif A, produisant un amplificat de taille T (T paires de base), marqué avec un marqueur M (par exemple un fluorochrome de couleur C), et
- Un couple d'amorces modifiées capable d'amplifier spécifiquement le motif A, produisant un amplificat de taille T±1 paire de bases, marqué avec un marqueur M (par exemple un fluorochrome de couleur C) ou avec un marqueur M' différent de M (par exemple un fluorochrome de couleur C' distincte de C).

Dans ce cas, il est par exemple possible d'utiliser un couple d'amorces modifiées dans lequel une des amorces contient une base de plus ou de moins par rapport à l'amorce équivalente du couple de référence.

Bien que ces paramètres ne changent pas la fiabilité du kit de l'invention, il est à noter que :
- La taille T-1 pb dans la couleur C est de préférence à éviter à cause des épaulements.
- La taille T-4pb dans la couleur C est de préférence à éviter à cause du phénomène de stutter.
- La taille T dans la couleur différente de C est de préférence à éviter à cause du phénomène de pull-up.

Outre ce 2-AMC, ce kit de PCR peut contenir tout réactif utilisable pour réaliser une PCR, à savoir une polymérase Taq recombinante, un tampon pour maintenir le pH stable dans le milieu réactionnel (typiquement du Tris-HCl), dNTPs, du MgCl2, etc.

### Pour n=3, le kit de l'invention contient un 3-AMC.

Dans un autre mode de réalisation particulier, le kit de l'invention contient un 3-AMC. Ceci permet de maximiser le nombre d'informations lues sur l'électrophorégramme tout en limitant le nombre d'erreur de lecture.

Dans ce cas, le kit de l'invention contient, pour chaque motif d'intérêt à identifier sur un acide nucléique, trois couples d'amorces spécifiques dudit motif, lesdits couples générant trois amplificats différenciables par leur taille et/ou par leur marquage.

Dans un mode de réalisation particulier, le kit de l'invention contient un couple d'amorces de référence capable d'amplifier spécifiquement le motif A, produisant un amplificat de taille T (T paires de base), marqué avec un marqueur M (par exemple un fluorochrome de couleur C), et au moins deux couples d'amorces choisis parmi :
- un couple d'amorces modifiées capable d'amplifier spécifiquement le motif A, produisant un amplificat ayant une taille T' différente de T, ledit amplificat étant marqué avec un marqueur M (par exemple un fluorochrome de couleur C),
- un couple d'amorces modifiées capable d'amplifier spécifiquement le motif A, produisant un amplificat ayant une taille T, ledit amplificat étant marqué avec un marqueur M' différent de M (par exemple un fluorochrome de couleur C' différente de C), et
- un couple d'amorces modifiées capable d'amplifier spécifiquement le motif A, produisant un amplificat ayant une taille T' différente de T, ledit amplificat étant marqué avec un marqueur M' différent de M (par exemple un fluorochrome de couleur C' différente de C).

De préférence, la taille T' de l'amplificat diffère de T d'une, de deux, de trois, voire de quatre paires de bases (en plus ou en moins).

Dans le cas plus particulier où T' diffère de T d'une paire de bases, le kit de l'invention contient un couple d'amorces de référence capable d'amplifier spécifiquement le motif A, produisant un amplificat de taille T (T paires de base), marqué avec un marqueur M (par exemple un fluorochrome de couleur C), et au moins deux couples d'amorces choisis parmi :
- un couple d'amorces modifiées capable d'amplifier spécifiquement le motif A, produisant un amplificat ayant une taille T±1 paire de bases, ledit amplificat étant marqué avec un marqueur M (par exemple un fluorochrome de couleur C),
- un couple d'amorces modifiées capable d'amplifier spécifiquement le motif A, produisant un amplificat ayant une taille T, ledit amplificat étant marqué avec un marqueur M' différent de M (par exemple un fluorochrome de couleur C' différente de C), et
- un couple d'amorces modifiées capable d'amplifier spécifiquement le motif A, produisant un amplificat ayant une taille T±1 paire de bases, ledit amplificat étant marqué avec un marqueur M' différent de M (par exemple un fluorochrome de couleur C' différente de C).

En particulier, le kit de l'invention peut contenir:
- Un couple d'amorces de référence capable d'amplifier spécifiquement le motif A, produisant un amplificat de taille T, marqué avec un marqueur M (par exemple un fluorochrome de couleur C),
- Un couple d'amorces modifiées capable d'amplifier spécifiquement le motif A, produisant un amplificat de taille T, marqué avec un marqueur M' différent de M (par exemple un fluorochrome de couleur C' distincte de C), et
- Un couple d'amorces modifiées capable d'amplifier spécifiquement le motif A, produisant un amplificat de taille T+1 paire de bases, marqué avec un marqueur M (par exemple un fluorochrome de couleur C).

Dans un autre mode de réalisation particulier, le kit de l'invention peut contenir :
- Un couple d'amorces de référence capable d'amplifier spécifiquement le motif A, produisant un amplificat de taille T, marqué avec un marqueur M (par exemple un fluorochrome de couleur C),
- Un couple d'amorces modifiées capable d'amplifier spécifiquement le motif A, produisant un amplificat de taille T+1 paire de bases, marqué avec un marqueur M (par exemple un fluorochrome de couleur C), et
- Un couple d'amorces modifiées capable d'amplifier spécifiquement le motif A, produisant un amplificat de taille T±1 paire de bases, marqué avec un marqueur M' différent de M (par exemple un fluorochrome de couleur C' distincte de C).

Outre ce 3-AMC, ce kit de PCR peut contenir tout réactif utilisable pour réaliser une PCR, à savoir une polymérase Taq recombinante, un tampon pour maintenir le pH stable dans le milieu réactionnel (typiquement du Tris-HCl), dNTPs, du MgCl2, etc.

### Détection d'un motif d'intérêt grâce au kit de l'invention

Un **n-ALC** est, au sens de la présente invention, le résultat visible de la détection d'un n-AMC dans un système à deux dimensions. Plus précisément, dans le cas d'une électrophorèse capillaire, il s'agit donc d'un groupe de n pics détectés sur l'électrophorégramme, apparaissant aux alentours de la taille T attendue pour l'amplificat. Chaque n-ALC révèle avec une très forte probabilité la présence (ou l'absence) du motif d'intérêt dans l'échantillon d'acide nucléique. Cet n-ALC permet notamment de caractériser objectivement quel(s) allèle(s) est (sont) porté(s) par l'individu testé.

Dans un second aspect, la présente invention vise une méthode permettant de détecter au moins un motif d'acide nucléique d'intérêt dans un échantillon biologique, à partir des n-ALC obtenus en utilisant le kit de l'invention. Naturellement, cette méthode nécessite d'utiliser autant de n-AMC que de locus d'intérêt.

Cette méthode utilise donc le kit de l'invention tel que décrit ci-dessus, dans lequel le nombre de n-AMC est ajusté au nombre de motifs à détecter.

Dans un mode de réalisation, la méthode permettant de détecter au moins un motif d'acide nucléique d'intérêt dans un échantillon biologique est caractérisée en ce qu'elle utilise le kit tel que défini ci-dessus et en ce que l'on détecte ledit motif d'acide nucléique d'intérêt lorsque l'on détecte, pour ledit motif, au moins un amplificat par couple d'amorces spécifiques dudit motif utilisé.

Plus précisément, la présente invention comprend donc les étapes suivantes :
a) Obtention d'un échantillon biologique contenant des acides nucléiques,
b) Mise en contact de l'échantillon biologique avec les couples d'amorces des n-AMC décrits ci-dessus, et des réactifs nécessaires à l'amplification desdits acides nucléiques,
c) Amplification desdits acides nucléiques dans les conditions appropriées,
d) Détection des amplificats obtenus au moyen de leur taille et/ou de leur marquage ou d'un système de détection à deux dimensions.
e) Génération d'un profil génétique en fonction des amplificats détectés à l'étape d).

Par « **échantillon biologique** », on entend ici tout échantillon susceptible de contenir des acides nucléiques, notamment un échantillon contenant des cellules (animales, humaines, végétales, microbiennes, etc). De préférence, dans le cadre d'une application en sciences forensiques, il s'agit d'un échantillon de sang, et, plus particulièrement, d'un échantillon de sérum ou de plasma prélevé sans étape invasive auprès d'un individu (humain ou animal). Il est également possible d'utiliser un échantillon de salive, de sperme, de cheveux, ou d'urine. Il est enfin possible d'utiliser des traces de contact (cellules de peau par exemple).

Dans des cas particuliers où la quantité d'acides nucléiques est trop faible dans l'échantillon prélevé ou lorsque lesdits acides nucléiques sont contenus dans des cellules, il est possible de rajouter au préalable de l'étape b) une étape d'extraction et/ou de purification et/ou de concentration desdits acides nucléiques. Toute technique connue de l'homme du métier à cet effet peut alors être utilisée (extraction Chelex, papier FTA, extraction en phase solide basée sur des colonnes de silices ou des billes magnétiques.). De telles techniques sont par exemple décrites dans Sambrook et al. (Molecular cloning: a laboratory manual, Cold Spring Harbor Laboratory; 3rd edition, 2001). En règle générale, la méthode de l'invention est réalisable sur un échantillon contenant une faible quantité d'ADN (par exemple 0.1 - 0.5ng d'ADN). Lorsque de telles étapes de purification ou de concentration sont réalisées, l'échantillon biologique servant à l'étape b) est bien évidemment celui ayant été purifié et/ou concentré.

A cet échantillon biologique est ajouté, au cours d'une seconde étape, un nombre adéquat de n-AMC (autant de n-AMC que de motifs à détecter), ainsi que les réactifs indispensables pour générer les amplificats attendus (une polymérase Taq recombinante, du Tris-HCl, des dNTPs, du MgCl2, etc.).

La méthode de l'invention contient ensuite une étape d'amplification c) au cours de laquelle une réaction de polymérisation en chaîne (PCR) est réalisée dans les conditions habituellement utilisées par l'homme du métier (Butler M. et al, Fundamentals of Forensic DNA typing, 2009). Plusieurs techniques alternatives à la PCR classique peuvent également être utilisées dans cette étape. Ces techniques sont par exemple la SDA (Strand Displacement Amplification), la technique TAS (Transcription-based Amplification System), la technique 3SR (Self-Sustained Sequence Replication), la technique NASBA (Nucleic Acid Sequence Based Amplification), la technique TMA (Transcription Mediated Amplification), la technique LCR (Ligase Chain Reaction), la technique de RCR (Repair Chain Reaction), la technique CPR (Cycling Probe Reaction), ou encore la technique d'amplification à la Q-béta-réplicase. De manière générale, il est possible d'utiliser dans cette étape toute technique aboutissant à l'amplification de l'acide nucléique cible. L'homme du métier saura identifier quels réactifs sont requis dans le milieu réactionnel, outre les n-AMCs de l'invention, pour générer les amplificats attendus.

A la suite de cette étape d'amplification, les amplificats produits sont détectés au moyen d'un système de détection à deux dimensions (par exemple taille / couleur). De préférence, ce système de détection est l'électrophorèse ou la chromatographie. De manière encore plus préférée, le système de détection est l'électrophorèse capillaire.

Enfin, les signaux correspondant aux amplificats marqués sont analysés en vue de conclure quant à la présence ou à l'absence des motifs d'intérêt dans l'échantillon.

Cette étape d'analyse se fait de préférence de manière automatique, par exemple à l'aide d'un logiciel capable de reconnaitre les signaux (ou les groupes de n pics ou les n-ALC) et les attribuer à un phénotype (selon l'une des deux méthodes d'analyse décrite ci-dessous).

La présente invention propose deux méthodes différentes pour analyser les signaux / groupes de n pics / n-ALC obtenus par la méthode de l'invention et conclure sur la présence du motif d'intérêt en conséquence.

### Première méthode d'analyse (dite « à dictionnaire ») :

Cette première méthode d'analyse nécessite la construction ou l'obtention, au préalable de l'analyse, d'un « dictionnaire » ou d'un « catalogue » recensant, pour chaque motif d'intérêt, tous les n-ALC qu'il est en théorie possible de détecter à l'aide des n-AMCs utilisés.

En d'autres termes, il convient d'établir - ou d'acquérir - la liste de toutes les combinaisons de signaux attendues pour chaque n-AMC choisi. Cette liste d'n-ALC constitue le dictionnaire des allèles valides. Elle est par exemple réalisée à partir des listes classiquement fournies dans les kits commerciaux (PowerPlex, etc.). Ces combinaisons sont aisément identifiables, pour chaque motif d'intérêt, une fois que les amorces de référence et les amorces modifiées ont été choisies, puisque ce choix détermine la taille de l'amplificat attendue en présence du motif d'intérêt vs. en son absence, ainsi que le marquage attendu pour chaque type d'amplificat.

Par exemple, pour détecter les 425 allèles possibles requis par la réglementation américaine, ledit dictionnaire recensera 425 n-ALCs. En outre, pour détecter les 552 allèles possibles requis par les réglementations européenne et américaine, ledit dictionnaire recensera 552 n-ALCs.

Pour obtenir la liste des motifs réellement présents dans l'échantillon, on compare ensuite toutes les combinaisons de n pics détectés pour chaque n-ALC de l'échantillon avec les n-ALC attendus pour ces motifs (i .e., ceux recensés au préalable dans la liste). Cette liste d'n-ALC constitue le dictionnaire des allèles valides.

La probabilité d'obtenir un n-ALC valide par cette méthode est 1-X/ Cⁿ_{R}, où X est le nombre de motifs à détecter, R est le nombre de tailles différentes détectables (toutes couleurs confondues) et n est le nombre de couples d'amorces utilisé pour chaque motif.

Dans la mesure où chaque canal de couleur peut produire autant de pics que de tailles de chaine possibles (en bp), il est possible de calculer que, dans un système classique contenant cinq canaux de couleurs (tel que ceux proposés par Global Filer), le nombre de pics détectables sur l'électrophorégramme est de 1850.

Ainsi, pour un tel système, R=1850 et il sera donc possible de générer Cⁿ₁₈₅₀ n-ALCs. Tous ayant une probabilité identique d'exister sur l'électrophorégramme en tant que signal analogique, la probabilité qu'un n-ALC soit valide / attendu est donc de 1-425/Cⁿ₁₈₅₀ (système américain) ou 1-552/Cⁿ₁₈₅₀ (systèmes américain et européen). Dans le cas où on utilise des 3-AMCs, la probabilité qu'une combinaison détectée soit un n-ALC valide est de 1-(425/C³₁₈₅₀) = 1-4,034x10⁻⁷ soit **99.99996** % (système américain) ou de 1-(552/C³₁₈₅₀) soit **99.99995** % (systèmes américain et européen).

Avec cette méthode basée sur un « dictionnaire », chaque combinaison valide identifiée permet de conclure avec certitude que le motif d'intérêt était présent dans l'échantillon testé. Pour résumer, il est proposé ici une méthode dans laquelle, pour détecter ledit motif nucléique d'intérêt, on compare toutes les combinaisons de n pics détectés dans l'échantillon pour chaque n-ALC, avec des n-ALC attendus contenus dans une liste préalablement établie.

Dans un mode de réalisation préféré, le kit de l'invention contient, outre les éléments définis ci-dessus, un document décrivant l'ensemble des n-ALCs attendus dans un échantillon (le « catalogue » ou le « dictionnaire » tel que décrit ci-dessus), qui permettra d'identifier à quels motifs correspondent les n-ALC détectés.

### Seconde méthode d'analyse (dite « à motif») :

La première méthode d'analyse, décrite ci-dessus, permet de détecter la présence d'un motif d'intérêt dont la structure est attendue (par exemple, un nombre de répétitions donné au sein d'un STRs, qui avait été recensé dans le « dictionnaire »). Cependant, dans la mesure où le « dictionnaire » ne peut pas être exhaustif quant à l'ensemble des motifs existants dans la nature, il peut exister des situations où le motif qui est détecté ne correspond à aucun de ceux recensés.

En particulier, cette première méthode ne permet pas d'identifier l'existence de motifs qui seraient spécifiques à un individu, et, de ce fait, non recensés dans le « dictionnaire » (par exemple un microvariant rare).

La détection de ces motifs rares est problématique avec les méthodes actuellement utilisées en sciences forensiques. En effet, il est parfois difficile de savoir s'ils sont de vrais micro-variants ou si le signal détecté correspond en fait à un pic ayant mal migré.

Pour améliorer ce point, l'invention propose une seconde méthode permettant d'analyser les n-ALCs obtenus par la méthode de l'invention utilisant les n-AMCs, en vue notamment de détecter la présence de motifs rares au sein d'un échantillon.

Cette seconde méthode s'appuie sur le fait que chaque groupe de n pics forme un n-ALC spécifique. Chaque n-ALC détecté valide correspond donc au motif d'intérêt cherché (par exemple, pour un 3-AMC tel que décrit ci-dessus, un n-ALC peut être : la taille T sur la couleur C, la taille T+1 sur la couleur C et la taille T-1 sur la couleur C-1).

D'une manière générale la probabilité d'obtenir un n-ALC valide avec cette méthode « à motifs » est 1-((1/R)^{∗}(1/R-1)^{∗}(...)^{∗}(1/R-(n-1))), R étant le nombre de tailles différentes détectables (toutes couleurs confondues) et n étant le nombre de couples d'amorces utilisé pour chaque motif.

Pour un système contenant 1850 possibilités de pics sur l'électrophorégramme, la probabilité qu'une combinaison détectée corresponde à un n-ALC valide est de 1 - (1^{∗}(1/1849)^{∗}(1/1848)) = **99,99997 %.**

Ainsi, avec cette seconde méthode, la présence d'un motif d'intérêt peut être identifiée avec certitude. Il est également possible de mesurer la taille du motif d'intérêt.

Pour résumer, il est proposé ici une méthode dans laquelle, pour détecter ledit motif nucléique d'intérêt, on met en oeuvre les étapes suivantes :
a) détection de tous les n-ALC valides dans l'échantillon testé,
b) attribution de chaque n-ALC valide à un motif d'intérêt recherché.

L'utilisation de l'une ou l'autre de ces deux méthodes d'analyse présente l'avantage que les pics liés à des artefacts ne formeront pas d'n-ALC valides, et ne pourront donc pas être confondus avec le motif d'intérêt. Ainsi, chaque n-ALC valide détecté (i.e., chaque combinaison de signaux générés par un n-AMC) permet de conclure que le motif d'intérêt est présent dans l'échantillon (méthode « du dictionnaire ») ou qu'un variant de ce motif est présent dans l'échantillon (méthode « à motif »).

Il est à noter que les deux méthodes d'analyse sont cumulables et peuvent être réalisées successivement ou concomitamment.

De plus, elles peuvent être réalisées de manière automatique.

Plus généralement, la méthode de l'invention est caractérisée en ce que l'étape de détection et de génération d'un profil génétique d) et e), et le cas échéant les étapes d'amplification et de mise en contact, sont réalisées de façon automatique.

Dans un mode de réalisation préféré, le kit de l'invention contient des n-AMCs avec n>2. En effet, dans ce cas, chaque n-ALC dispose d'une portion de code agissant comme **« code correcteur d'erreur »**, permettant la détection du motif d'intérêt même s'il est peu différenciable du bruit.

Lorsque n>2, il est en effet possible de créer des n-ALCs contenant une combinaison unique de n-1 éléments (« n-1ALCs »), qui peut être retrouvée dans la liste des n-ALC attendues.

Avec la méthode d'analyse « à dictionnaire », il suffit donc d'avoir n-1 éléments d'un n-ALC pour identifier un n-ALC (et donc le motif d'intérêt lui correspondant). La probabilité d'avoir une information valide est de 1 - (X/ Cⁿ⁻¹_{R}), où X est le nombre de motifs d'intérêt à détecter, R est le nombre de tailles différentes détectables (toutes couleurs confondues) et n est le nombre de couples d'amorces utilisé pour chaque motif. Pour un système contenant 1850 possibilités de pics sur l'électrophorégramme, la probabilité qu'une combinaison corrigée (via un code correcteur) soit valide est, pour le système américain, 1-(425/C²₁₈₅₀) = 1-2,485x10⁻⁴ = 99.9752 %, et, pour les systèmes américains et européens, 1-(552/C²₁₈₅₀) = 99.9677 %.

Avec la méthode d'analyse « à motif », la probabilité d'avoir une information valide est 1-((1/R)^{∗}(1/R-1)^{∗}(...)^{∗}(1/R-(n-2))) où R est le nombre de tailles différentes détectables (toutes couleurs confondues) et n est le nombre de couples d'amorces utilisé pour chaque motif. Pour un système contenant 1850 possibilités de pics sur l'électrophorégramme, la probabilité qu'une combinaison corrigée (via un code correcteur) soit valide est de 1 - (1^{∗}(1/1849)) soit 99.946 %.

Lors de l'analyse des signaux obtenus par la méthode de l'invention, la séparation signal / bruit est classiquement réalisée par seuillage à 10σ et détection de la forme du pic. Cependant, ces méthodes ne permettent pas de produire une probabilité objective de la réalité de l'information lue. Le seuillage à 10σ pourrait en produire une, mais la distribution du bruit ne suit pas une loi connue (type loi normale) sur un électrophorégramme. De plus, la détection de la forme du pic se base sur des descriptions et des observations empiriques de la forme que doit avoir un pic.

Dans le cadre de la présente invention, vu la faible probabilité d'obtenir un faux n-ALC, il est possible de faire une sélection des signaux analysés bien en dessous de ce qui est fait actuellement (seuillage à 10σ et détection de la forme du pic). En particulier, un seuillage à 3σ suffit, dans le cadre des méthodes de l'invention, pour bien séparer le signal du bruit tout en produisant plus d'informations que les méthodes actuelles. En effet le seuillage à 3σ au lieu de 10σ apporte une acceptation du bruit non significative par rapport à la probabilité qu'un n-ALC soit valide.

Concernant le seuillage à 3σ, il est préférable, avant la lecture des n-ALC d'utiliser un filtre annulateur d'écho sur le signal de l'éléctrophorégramme. L'écho recherché correspond au stutter et épaulement. Les méthodes d'annulation d'écho sont connues de l'homme du métier concernant le traitement du signal (téléphonie, communications réseaux,...). Les méthodes à employer sont les plus simples : les paramètres de l'écho sont fixes : écho à -1 pb pour l'épaulement et écho à -4 pb pour le stutter. Il est également possible de chercher des variations de fréquence dans les n-ALCs plutôt que des pics (le bruit a une fréquence relativement constante, diminuant avec la longueur des acides nucléiques). Ceci est particulièrement préférable lorsque les polynucléotides détectés sont de grande taille.

La recherche de pics et de variations de fréquence sont cumulables.

Lors de l'analyse des signaux obtenus par la méthode de l'invention, il existe des cas pour lesquelles des portions d'n-ALC sont recouverts (totalement ou partiellement) par d'autres n-ALC. Ceci n'est pas un problème, puisque la méthode de l'invention requiert de tenir compte de toutes les combinaisons/motifs de n pics trouvés. Seuls les n-ALC valides seront conservés avec les probabilités associées indiquées plus haut.

### Description des figures

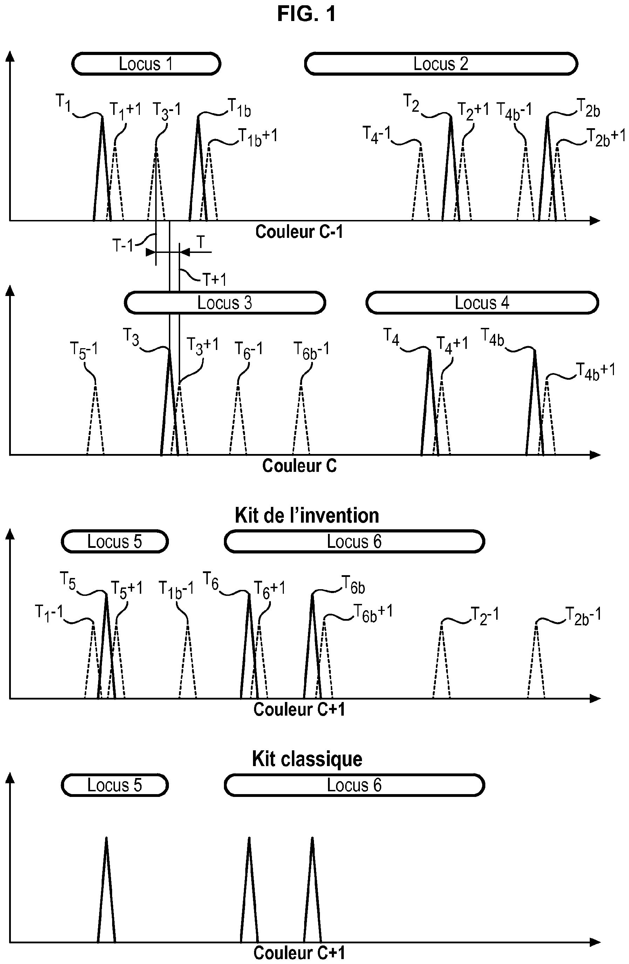

La **figure 1** illustre un exemple d'électrophorégramme pouvant être obtenu avec le kit de PCR de l'invention en utilisant un 3-AMC au sens de l'invention (3 amorces permettant de détecter chaque allèle du locus d'intérêt), en utilisant 3 couleurs (C-1, C et C+1). Les traits pleins représentent les pics obtenus classiquement avec les kits commerciaux (voir également l'électrophorégramme obtenu avec un « kit classique », dernier panel en bas de la figure). Les traits pleins et pointillés représentent les pics obtenus en utilisant le kit de PCR de l'invention (Tx= pic correspondant à un amplificat de taille T pour l'allèle du locus X, Txb= pic correspondant à l'amplificat du 2^{ème} allèle du locus X si l'individu est hétérozygote, Tx+1= pic correspondant à l'amplificat de taille T+1 paire de bases pour l'allèle du locus X, Tx-1= pic correspondant à l'amplificat de taille T-1 paire de bases pour l'allèle du locus X).x est ici un entier compris entre 1 et 6.

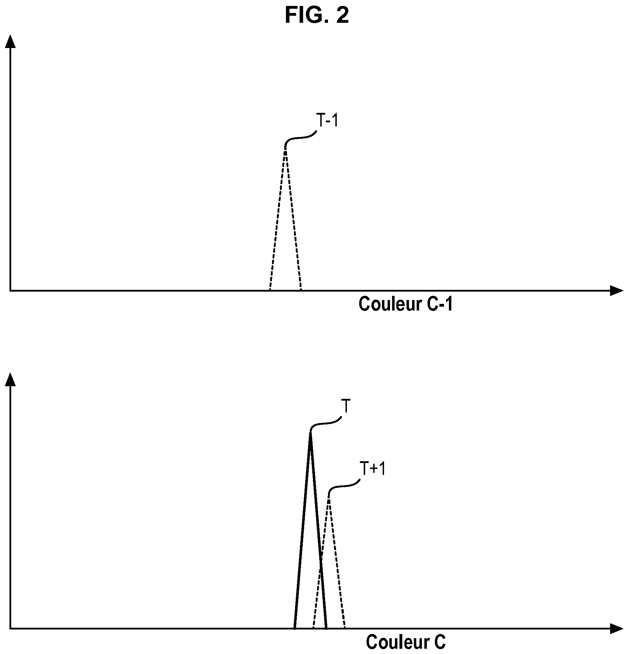

La **figure 2** illustre un exemple de réalisation de la méthode à motif décrite dans la présente demande, en utilisant le kit de PCR de l'invention avec n=3, le motif recherché étant la combinaison : T/C, T+1/C et T-1/C-1. Les deux graphiques représentent les électrophorégrammes obtenus pour la couleur C-1 (en haut) et C (en bas). Le trait plein représente le pic obtenu classiquement avec un kit commercial (correspondant à un amplificat de taille T), les traits pointillés représentent ceux obtenus avec les marqueurs de l'invention (générant des amplificats de taille T-1 et T+1, sur les couleurs C-1 et C respectivement).

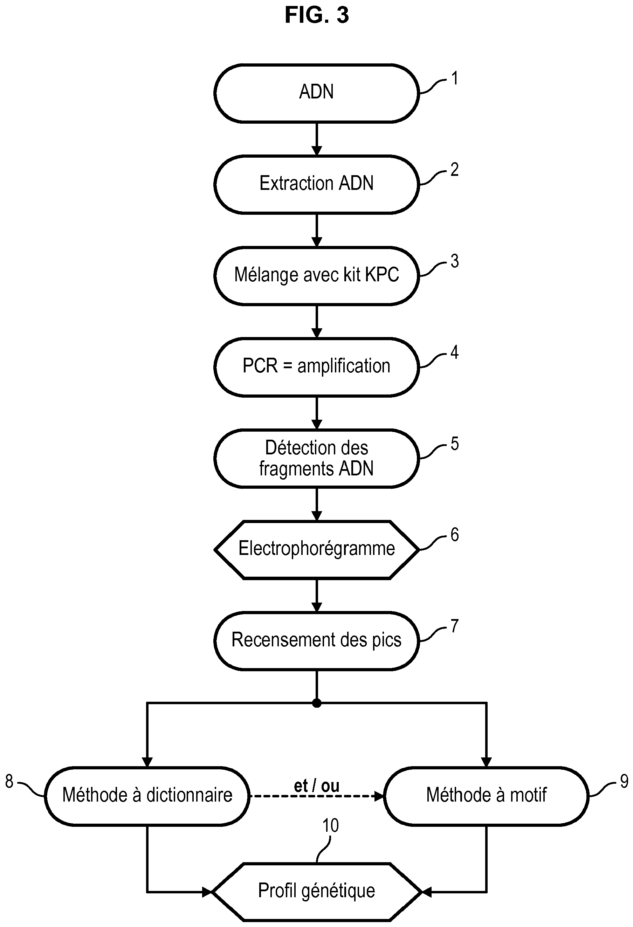

La **figure 3** illustre un logigramme représentant la technique de détection d'un profil génétique selon la présente invention. A partir d'un échantillon d'un individu contenant de l'ADN (1), l'ADN est extrait avec des techniques classiques (2) puis mis en contact avec les composants (dNTPs, amorces, polymérase, etc.) du kit PCR de l'invention (3). Une PCR est réalisée (4) puis l'échantillon contenant les amplificats est analysé par électrophorèse capillaire (5), générant un électrophorégramme (6) sur lequel les pics correspondant aux amplificats marqués sont recensés (7). L'analyse de ces pics peut être réalisée soit par la méthode à dictionnaire (8), soit par la méthode à motif (9), voire par les deux méthodes cumulativement, de sorte à détecter le profil génétique de l'individu (10). Cette détection est plus fiable que les techniques actuelles et peut se faire de façon automatique.

## Revendications

1. Kit pour PCR comprenant au moins n couples d'amorces de PCR (n-AMC) spécifiques à un même motif d'intérêt à identifier sur un acide nucléique, avec n égal ou supérieur à 2, lesdites amorces au sein de chaque couple s'hybridant spécifiquement de part et d'autre dudit motif d'intérêt,
**caractérisé en ce qu'**il contient :
- un couple d'amorces de référence spécifique dudit motif d'intérêt, portant un marqueur M, et
- au moins un couple d'amorces modifiées spécifique dudit motif d'intérêt,
dans lequel :
i) au moins une des amorces modifiées contient X base(s) de plus ou de moins par rapport à l'amorce équivalente dans le couple des amorces de référence, X étant un entier compris entre 1 et 10, ou
ii) les amorces modifiées ont une région d'hybridation sur l'acide nucléique d'intérêt décalée de X base(s) par rapport à celle des amorces de référence, X étant un entier compris entre 1 et 10,
et/ou
iii) au moins une des amorces modifiées porte un marqueur différent du ou des marqueur(s) porté(s) par les amorces de référence,
de sorte à générer, pour chaque motif, au moins n amplificats de PCR différenciables par leur taille et/ou par leur marquage, générant ainsi une redondance de l'information permettant de coder ledit motif d'intérêt.

2. Kit pour PCR selon la revendication 1, **caractérisé en ce que** X est un entier compris entre 1 et 4.

3. Kit selon l'une des revendications 1 à 2, contenant :
- un couple d'amorces de référence adapté pour amplifier spécifiquement le motif A, produisant un amplificat de taille T (T paires de base), marqué avec un marqueur M, et
- un couple d'amorces modifiées adapté pour amplifier spécifiquement le motif A, produisant un amplificat de taille T' différent de T par une, deux, trois ou quatre paires de base en plus ou en moins, marqué avec un marqueur M' différent de M.

4. Kit selon l'une des revendications 1 à 3, comprenant au moins n couples d'amorces n-AMC, avec n strictement supérieur à 2.

5. Kit selon l'une des revendications précédentes, contenant, pour chaque motif d'intérêt à identifier sur un acide nucléique, trois couples d'amorces spécifiques dudit motif.

6. Kit selon l'une des revendications précédentes, contenant un couple d'amorces de référence capable d'amplifier spécifiquement le motif A, produisant un amplificat de taille T (T paires de base), marqué avec un marqueur M, et au moins deux autres couples d'amorces choisis parmi :
- un couple d'amorces modifiées adapté pour amplifier spécifiquement le motif A, produisant un amplificat ayant une taille T' différent de T par une, deux, trois ou quatre paires de base en plus ou en moins, ledit amplificat étant marqué avec un marqueur M,
- un couple d'amorces modifiées adapté pour amplifier spécifiquement le motif A, produisant un amplificat ayant une taille T, ledit amplificat étant marqué avec un marqueur M' différent de M, et
- un couple d'amorces modifiées adapté pour amplifier spécifiquement le motif A, produisant un amplificat ayant une taille T' différent de T par une, deux, trois ou quatre paires de base en plus ou en moins, ledit amplificat étant marqué avec un marqueur M' différent de M.

7. Kit selon l'une des revendications précédentes, dans lequel tous les couples d'amorces sont pré-mélangés dans un contenant unique.

8. Méthode permettant de détecter au moins un motif d'acide nucléique d'intérêt dans un échantillon biologique, **caractérisée en ce qu'**elle utilise un kit selon l'une des revendications 1-7 et **en ce que** l'on détecte ledit motif d'acide nucléique d'intérêt lorsque l'on détecte, pour ledit motif, au moins un amplificat par couple d'amorces spécifiques dudit motif utilisé.

9. Méthode selon la revendication 8, comprenant les étapes suivantes :
a) Mise en contact d'un échantillon de sang, de salive, de sperme, de cheveux, d'urine, de sérum ou de plasma contenant des acides nucléiques avec les couples d'amorces décrits dans les revendications 1-7 et des réactifs nécessaires à l'amplification desdits acides nucléiques,
b) Amplification desdits acides nucléiques dans les conditions appropriées,
c) Détection des amplificats obtenus au moyen de leur taille et/ou de leur marquage, de préférence par électrophorèse capillaire,
d) Génération d'un profil génétique en fonction des amplificats détectés à l'étape c).

10. La méthode selon l'une quelconque des revendications 8 à 9, dans laquelle ledit motif d'intérêt est un STR.

11. Méthode selon la revendication 8, dans laquelle, pour détecter ledit motif nucléique d'intérêt, on compare toutes les combinaisons de n pics détectés dans l'échantillon pour chaque groupe de n pics obtenus, avec des groupes de n pics attendus contenus dans une liste préalablement établie.

12. Méthode selon la revendication 8 prise seule ou en combinaison avec la revendication 11, dans laquelle, pour détecter ledit motif nucléique d'intérêt, on met en oeuvre les étapes suivantes :
a) détection de tous les groupes de n pics valides dans l'échantillon testé,
b) attribution de chaque groupe de n pics valide à un motif d'intérêt recherché.

13. Méthode selon la revendication 9 prise seule ou en combinaison avec l'une des revendications 10 à 12, **caractérisée en ce que** l'étape de détection et de génération d'un profil génétique c) et d), et le cas échéant les étapes d'amplification et de mise en contact, sont réalisées de façon automatique.

## Patentansprüche

1. Kit für PCR, umfassend mindestens n PCR-Primerpaare (n-AMC), die für ein selbes, auf einer Nukleinsäure zu identifizierendes Interessensmotiv spezifisch sind, mit n gleich oder größer als 2, wobei die Primer innerhalb jedes Paares spezifisch beiderseits des Interessensmotivs hybridisieren,
**dadurch gekennzeichnet, dass** es enthält:
- ein für das Interessensmotiv spezifisches Referenz-Primerpaar, das einen Marker M trägt, und
- mindestens ein für das Interessensmotiv spezifisches Paar modifizierter Primer, wobei
i) mindestens einer der modifizierten Primer X Basen mehr oder weniger in Bezug auf den äquivalenten Primer im Referenzprimerpaar enthält, wobei X eine Ganzzahl ist, die zwischen 1 und 10 liegt, oder
ii) die modifizierten Primer eine Hybridisierungsregion auf der interessierenden Nukleinsäure haben, die um X Basen in Bezug auf die der Referenzprimer versetzt ist, wobei X eine Ganzzahl ist, die zwischen 1 und 10 liegt,
und/oder
iii) mindestens einer der modifizierten Primer einen Marker trägt, der sich von dem oder den Markern unterscheidet, der/die von den Referenzprimern getragen wird/werden,
so dass für jedes Motiv mindestens n PCR-Amplifikate erzeugt werden, die durch ihre Größe und/oder ihre Markierung unterscheidbar sind, wodurch eine Redundanz der Information erzeugt wird, die erlaubt, das Interessensmotiv zu codieren.

2. Kit für PCR nach Anspruch 1, **dadurch gekennzeichnet, dass** X eine Ganzzahl ist, die zwischen 1 und 4 liegt.

3. Kit nach einem der Ansprüche 1 bis 2, enthaltend:
- ein Referenzprimerpaar, das geeignet ist, spezifisch das Motiv A zu amplifizieren, wobei ein Amplifikat der Größe T (T Basenpaare) entsteht, markiert mit einem Marker M, und
- ein Paar modifizierter Primer, das geeignet ist, spezifisch das Motiv A zu amplifizieren, wobei ein Amplifikat der Größe T' entsteht, die sich von T durch ein, zwei, drei oder vier Basenpaare mehr oder weniger unterscheidet, markiert mit einem Marker M', der sich von M unterscheidet.

4. Kit nach einem der Ansprüche 1 bis 3, umfassend mindestens n Primerpaare n-AMC mit n streng größer als 2.

5. Kit nach einem der vorangehenden Ansprüche, enthaltend, für jedes auf einer Nukleinsäure zu identifizierendes Interessensmotiv, drei Paare spezifischer Primer des Motivs.

6. Kit nach einem der vorangehenden Ansprüche, enthaltend ein Referenzprimerpaar, das imstande ist, spezifisch das Motiv A zu amplifizieren, wobei ein Amplifikat der Größe T (T Basenpaare) entsteht, markiert mit einem Marker M, und mindestens zwei andere Primerpaare, ausgewählt aus:
- einem Paar modifizierter Primer, das geeignet ist, spezifisch das Motiv A zu amplifizieren, wobei ein Amplifikat entsteht, das eine Größe T' hat, die sich von T durch ein, zwei, drei oder vier Basenpaare mehr oder weniger unterscheidet, wobei das Amplifikat mit einem Marker M markiert ist,
- einem Paar modifizierter Primer, das geeignet ist, spezifisch das Motiv A zu amplifizieren, wobei ein Amplifikat entsteht, das eine Größe T hat, wobei das Amplifikat mit einem Marker M' markiert ist, der sich von M unterscheidet, und
- einem Paar modifizierter Primer, das geeignet ist, spezifisch das Motiv A zu amplifizieren, wobei ein Amplifikat entsteht, das eine Größe T' hat, die sich von T durch ein, zwei, drei oder vier Basenpaare mehr oder weniger unterscheidet, wobei das Amplifikat mit einem Marker M' markiert ist, der sich von M unterscheidet.

7. Kit nach einem der vorangehenden Ansprüche, wobei alle Primerpaare in einem einzigen Behältnis vorgemischt werden.

8. Methode, die erlaubt, mindestens ein Nukleinsäure-Interessensmotiv in einer biologischen Probe zu ermitteln, **dadurch gekennzeichnet, dass** sie ein Kit nach einem der Ansprüche 1-7 verwendet und dass das Nukleinsäure-Interessensmotiv ermittelt wird, wenn für das Motiv mindestens ein Amplifikat je Paar spezifischer Primer des verwendeten Motivs ermittelt wird.

9. Methode nach Anspruch 8, umfassend die folgenden Schritte:
a) Inkontaktversetzen einer Blut-, Speichel-, Sperma-, Haar-, Urin-, Serum- oder Plasmaprobe, enthaltend Nukleinsäuren, mit den in den Ansprüchen 1-7 beschriebenen Primerpaaren und Reagenzien, die für die Amplifikation der Nukleinsäuren notwendig sind,
b) Amplifikation der Nukleinsäuren unter den geeigneten Bedingungen,
c) Ermitteln der erhaltenen Amplifikate mittels ihrer Größe und/oder ihrer Markierung, vorzugsweise durch Kapillarelektrophorese,
d) Erzeugen eines genetischen Profils in Abhängigkeit von den in Schritt c) ermittelten Amplifikaten.

10. Die Methode nach einem der Ansprüche 8 bis 9, wobei das Interessensmotiv ein STR ist.

11. Methode nach Anspruch 8, wobei zur Ermittlung des Nuklein-Interessensmotiv alle Kombinationen von n Pics, die in der Probe für jede Gruppe von n erhaltenen Pics ermittelt wurden, mit Gruppen von n erwarteten Pics verglichen werden, die in einer zuvor erstellten Liste enthalten sind.

12. Methode nach Anspruch 8, herangezogen allein oder in Kombination mit Anspruch 11, wobei zur Ermittlung des Nuklein-Interessensmotivs die folgenden Schritte durchgeführt werden:
a) Ermitteln aller bestätigter Gruppen von n Pics in der getesteten Probe,
b) Zuweisen jeder bestätigten Gruppe von n Pics einem gesuchten Interessensmotiv.

13. Methode nach Anspruch 9, herangezogen allein oder in Kombination mit einem der Ansprüche 10 bis 12, **dadurch gekennzeichnet, dass** der Schritt des Ermittelns und des Erzeugens eines genetischen Profils c) und d) und gegebenenfalls die Schritte der Amplifikation und des Inkontaktversetzens automatisch durchgeführt werden.

## Claims

1. PCR kit comprising at least n PCR primer pairs (n-AMC) specific to the same motif of interest to be identified on a nucleic acid, with n equal to or greater than 2, said primers within each pair hybridizing specifically on either side of said motif of interest,
**characterized in that** it contains:
- a reference primer pair specific to said motif of interest, carrying a label M, and
- at least one modified primer pair specific to said motif of interest,
wherein:
(i) at least one of the modified primers contains X base(s) more or fewer relative to the equivalent primer of the reference primer pair, X being an integer between 1 and 10, or
(ii) the modified primers have a hybridization region on the nucleic acid of interest shifted by X base(s) relative to that of the reference primers, X being an integer between 1 and 10,
and/or
(iii) at least one of the modified primers carries a label different from the label(s) carried by the reference primers,
so as to generate, for each motif, at least n PCR amplicons differentiable by their size and/or their labeling, thus generating redundancy of information allowing said motif of interest to be encoded.

2. PCR kit according to claim 1, **characterized in that** X is an integer between 1 and 4.

3. Kit according to one of claims 1 to 2, containing :
- a reference primer pair adapted to specifically amplify motif A, producing an amplicon of size T (T base pairs), labeled with a label M, and
- a modified primer pair adapted to specifically amplify motif A, producing an amplicon of size T' different from T by one, two, three or four base pairs more or fewer, labeled with a label M' different from M.

4. Kit according to one of claims 1 to 3, comprising at least n primer pairs n-AMC, with n strictly greater than 2.

5. Kit according to one of the preceding claims, containing, for each motif of interest to be identified on a nucleic acid, three primer pairs specific to said motif.

6. Kit according to one of the preceding claims, containing a reference primer pair capable of specifically amplifying motif A, producing an amplicon of size T (T base pairs), labeled with a label M, and at least two other primer pairs selected from :
- a modified primer pair adapted to specifically amplify motif A, producing an amplicon having a size T' different from T by one, two, three or four base pairs more or fewer, said amplicon being labeled with a label M,
- a modified primer pair adapted to specifically amplify motif A, producing an amplicon having a size T, said amplicon being labeled with a label M' different from M, and
- a modified primer pair adapted to specifically amplify motif A, producing an amplicon having a size T' different from T by one, two, three or four base pairs more or fewer, said amplicon being labeled with a label M' different from M.

7. Kit according to one of the preceding claims, wherein all the primer pairs are premixed in a single container.

8. Method for detecting at least one nucleic acid motif of interest in a biological sample, **characterized in that** it uses a kit according to one of claims 1-7 and **in that** said nucleic acid motif of interest is detected when, for said motif, at least one amplicon per primer pair, specific to said motif used, is detected.

9. Method according to claim 8, comprising the following steps:
a) Contacting a sample of blood, saliva, semen, hair, urine, serum or plasma containing nucleic acids with the primer pairs described in claims 1-7 and reagents required to amplify said nucleic acids,
b) Amplifying said nucleic acids under suitable conditions,
c) Detecting the amplicons obtained by means of their size and/or their labeling, preferably by capillary electrophoresis,
d) Generating a genetic profile according to the amplicons detected in step c).

10. The method according to any one of claims 8 to 9, wherein said motif of interest is an STR.

11. Method according to claim 8, wherein, to detect said nucleic motif of interest, all the combinations of n peaks detected in the sample for each set of n peaks obtained are compared with the expected sets of n peaks contained in a list compiled beforehand.

12. Method according to claim 8 taken alone or in combination with claim 11, wherein, to detect said nucleic motif of interest, the following steps are carried out:
(a) detecting all the valid sets of n peaks in the sample tested,
(b) attributing each valid set of n peaks to a motif of interest sought.

13. Method according to claim 9 taken alone or in combination with one of claims 10 to 12, **characterized in that** the steps of detecting and generating a genetic profile, c) and d), and if need be the steps of amplifying and contacting, are carried out automatically.
